# EUROPEAN PATENT SPECIFICATION

(11) **EP 0 531 031 B1**
(45) Date of publication and mention of the grant of the patent: **02.11.1995**
(21) Application number: 92307687.1
(22) Date of filing: 21.08.1992
(51) Int. Cl.: A61K 31/13, A61K 31/095, A61K 31/19

(54) **Compositions for use in cancer therapy**
Zusammensetzungen für die Krebstherapie
Compositions pour utilisation dans la thérapie du cancer

(30) Priority: 06.09.1991 GB 9119081; 29.05.1992 GB 9211396
(43) Date of publication of application: 10.03.1993
(73) Proprietor: Holt, John Alfred Gorton, Perth, Western Australia 6009 (AU)
(72) Inventor: Holt, John Alfred Gorton, Perth, Western Australia 6009 (AU)
(74) Representative: Brown, David Leslie

(56) References cited:
- Dialog Abstract No. 00844513, 1991
- PHARMACOL. THER. vol. 46, no. 2, 1990, pages 243 - 271; G. AHLUWALIA ET AL.:'Metabolism and action of amino acid analog anti-cancer agents'
- NEUROSURGERY vol. 26, no. 2, 1990, pages 255 - 260; BODO E. LIPPITZ ET AL.: 'L-Buthionine-sulfoximine-mediated radiosensitization in experimental interstitial radiotherapy of intercerebral D-54 MG glioma xenografts in athymic mice"
- CANCER RESEARCH vol. 50, no. 4, 1990, pages 1251 - 1256; ISTVAN FEKETE ET AL.:'Rate of buthionine sulfoximine entry into brain and xenotransplanted humangliomas'
- CARCINOGENESIS vol. 10, no. 8, 1989, pages 1499 - 1503; MOHAMMED ATHAR ET AL.:'Evidence for the metabolism of tumor promoter organic peroxides into free radicals by human carninoma skin kerationcytes: an ESR-spin trapping study"

## Description

The present invention relates to compositions for use in a novel cancer therapy.

The use of ultra-high frequency (UHF) microwave electromagnetic radiation in human cancer therapy is known. For example, it is known that when a patient is exposed to such radiation prior to X-ray therapy the cancer cell kill can be increased by between about three and one hundred times compared with X-ray therapy alone.

The present invention is based on my surprising finding that by providing, at the time of administering the microwave radiation, an effective amount of one or more selected chemical agents in the vicinity of the cancer cells, the conventional subsequent X-ray or other cancer therapies can be avoided while yet retaining a remarkable degree of cancer cell destruction.

More particularly, the chemical agent used should be selected from non-toxic organic disulfides and optionally oxidising agents and organic sulfoximines. Mixtures of compounds of the same category, or different categories, may be used; in some cases different active agents may be administered sequentially, either for convenience or to prevent degradation or mutual pre-reaction of the agents. For example, when both organic disulfides and oxidising agents are used, the oxidising agent is normally administered before the organic disulfide to minimise degradation of the disulfide by reducing enzymes in the patient's body.

Without wishing to be bound by theory, it is believed that the chemical agent interferes with a redox cycle involving N,N'-[dithiobis[1-[(carboxymethyl)carbamoyl]ethylene]]diglutamine, which has the formula: 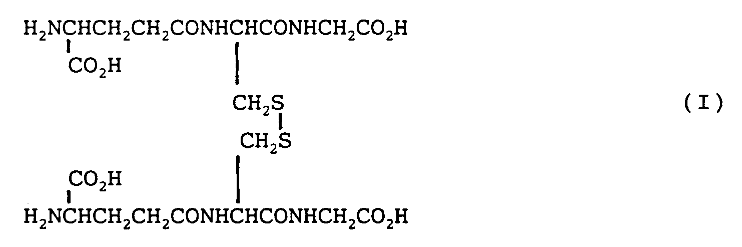
and glutathione, which has the formula: 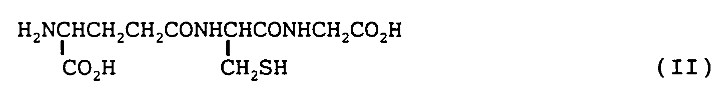
or with associated enzyme-catalysed reactions, which are believed to play an important part in the growth of cancerous cells.

According to a first aspect, therefore, the invention provides the use of a non-toxic organic disulfide having the general formula:

R-S-S-R¹ (III)

in which R and R¹, which may be the same or different, are selected from alkyl groups which may optionally be mono- or poly-substituted, in the preparation of a composition or compositions for use in a method of cancer therapy in which there is administered to a patient an effective amount of said non-toxic disulfide, and while said disulfide is present at a cancer site of the patient there is administered to the cancer site an effective dose of microwave electromagnetic radiation of frequency in the range of about 400-450MHz.

The expression "non-toxic" used herein refers to an acceptably low level of toxicity when a compound is present at an effective amount, and not necessarily to a complete absence of toxic effects. In particular, compounds which have only a temporary intoxicating effect and do no significant permanent harm, will be referred to and understood as "non-toxic" herein.

Included within the scope of the invention is the use of non-toxic precursors of the said agents, which are administered to the patient and are modified *in vivo* to provide the desired agent at the cancer site. For example, a thiol can be administered simultaneously with an oxidising agent to provide the corresponding disulfide and optionally an excess of the oxidising agent at the cancer site.

In the non-toxic organic disulfide, alkyl groups may suitably be selected from methyl, ethyl, propyl, n-butyl, s-butyl or t-butyl groups. Substituents of alkyl groups, when present, may be selected from any substituent atom(s) and group(s) whose presence as substituent does not significantly reduce the effectiveness of the agents in the therapy described and does not lead to intolerable side effects such as toxicity.

Examples of suitable substituents of R and/or R¹ in the compounds of formula III include halo (e.g. fluoro, chloro and bromo), nitro, amino, C₁₋₄alkylamino, di-C₁₋₄ alkylamino, hydroxy, C₁₋₄alkoxy, carboxy, (C₁₋₄alkyl)-carbonyl, (C₁₋₄alkoxy)-carbonyl, (C₁₋₄alkyl)-carbamoyl, carboxy-(C₁₋₄alkyl)-carbamoyl and C₂₋₅ alkanamido, and any salt, ester or other derivative forms thereof. Preferred substituents, when present, are selected from amino and carboxy.

Alkyl portions of any substituent of R and/or R¹ in compounds of formula III may optionally be mono- or poly-substituted by NH₂ and/or CO₂H and/or salt derivatives thereof.

A particularly preferred disulfide is cystine, which has the formula: 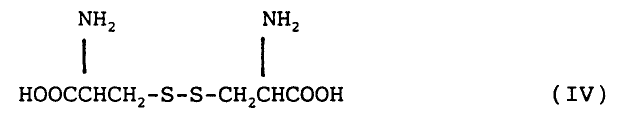
or a non-toxic salt thereof.

An optically active form of cystine, or alternatively an optically inactive (internally compensated) form or a racemic mixture, may be used. The laevorotatory form L-cystine is preferred by reason of its availability.

There may also be mentioned as a suitable organic disulfide penicillamine disulfide (3,3,3',3'-tetramethylcystine), which has the formula: 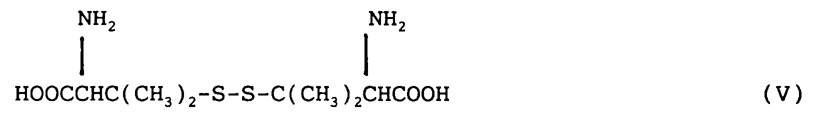
or a non-toxic salt thereof. An optically active form of penicillamine disulfide or a racemic mixture may be used.

There may optionally be used an oxidising agent preferably selected from organic peroxides and/or hydroperoxides of the general formulae

R³-O-O-R⁴ (VI)

and

R⁵-O-OH (VII)

respectively, in which R³, R⁴ and R⁵, which may be the same or different, are organic groups, most preferably selected from alkyl (e.g. methyl, ethyl, propyl, n-butyl, s-butyl or t-butyl) groups which may optionally be mono-or poly-substituted.

In the agents of formulae VI and VII as defined above, substituents of alkyl groups, when present, may be selected from any substituent atom(s) and group(s) whose presence as substituent does not significantly reduce the effectiveness of the agents in the therapy described and does not lead to intolerable side effects such as toxicity.

Examples of suitable substituents of R³, R⁴ and R⁵ include halo (e.g. fluoro, chloro and bromo), nitro, aryl (e.g. phenyl) and substituted aryl (e.g. aryl mono- or poly-substituted by alkyl, halo and/or nitro). Preferred substituents, when present, are aryl groups.

A particularly preferred oxidising agent is cumene hydroperoxide. Any of the hydroperoxides of cumene may be used. T-butyl hydroperoxide may also be particularly mentioned as an oxidising agent.

Further, there may optionally be used an organic sulfoximine of the general formula: 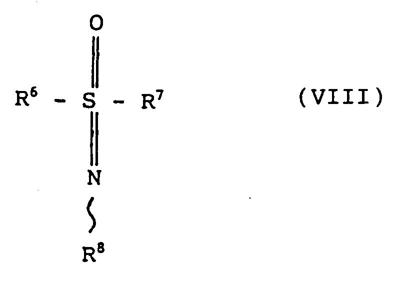
in which R⁶ and R⁷, which may be the same or different, are selected from alkyl (e.g. methyl, ethyl, propyl, n-butyl, s-butyl or t-butyl) groups which may optionally be mono- or poly-substituted, and R⁸ is hydrogen or an alkyl group, for example methyl, ethyl, propyl, n-butyl, s-butyl or t-butyl, which may optionally be mono- or poly-substituted.

In the agents of formula VIII as defined above, substituents of alkyl groups, when present, may be selected from any substituent atom(s) and group(s) whose presence as substituent does not significantly reduce the effectiveness of the agents in the therapy described and does not lead to intolerable side effects such as toxicity.

Examples of suitable substituents of R⁶, R⁷ and R⁸ include halo (e.g. fluoro, chloro and bromo), nitro, amino, C₁₋₄alkylamino, di-C₁₋₄alkylamino, hydroxy, C₁₋₄alkoxy, carboxy, (C₁₋₄alkyl)-carbonyl and (C₁₋₄alkoxy)-carbonyl, or any salt, ester or other derivative forms thereof. Preferred substituents, when present, are selected from amino and carboxy.

A particularly preferred sulfoximine is methionine sulfoximine, which has the formula: 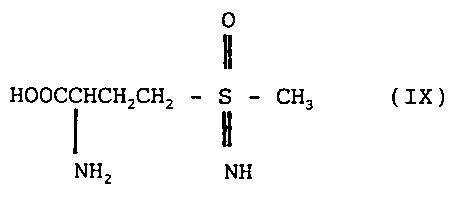
It has been found that certain organic alcohols, most particularly ethanol, can have beneficial effects when present in addition to the active agent(s) described above at the cancer site during the therapy. Such alcohols are not, however, effective *per se* at non-toxic dosages.

The composition form by which the active agent is administered to the patient may take any suitable form and employ any suitable conventional pharmaceutical carrier(s) or excipient(s). The composition form is preferably an aqueous solution suitable for intravenous drip or injection. Intravenous drip or injection is the preferred administration route by virtue of its rapid delivery of the active agent to the desired cancer site(s) with minimal degradation of the active agent.

The solution may if desired include additional components such as salts (e.g. sodium chloride), solubilising agents and the like. Oxidants such as hydrogen peroxide may be used in particular to prevent a disulfide splitting into its corresponding thiol(s).

The dose of chemical agent is selected according to the body weight of the patient, the tolerance of the patient to the active agent used, the ability of the active agent to remain at the cancer site or in the bloodstream without loss or degradation, the efficiency of the particular agent, the severity of the cancer to be treated, and the projected length of the overall treatment program.

An aqueous solution of L-cystine or penicillamine disulfide at a concentration of up to approximately 20 g/l (e.g. from about 10 to 20 g/l, suitably about 15 g/l) is convenient, enabling a daily injection volume between about 30 and 150 ml (e.g. about 50-60 ml), delivering a daily dose of up to approximately 3 g. Therapy using such a solution can produce an improvement after approximately 15 treatment days (typically over a few weeks) and is particularly effective on slower growing cancers.

To assist the passing of L-cystine into solution, a solubilising agent such as N-methyl-D-glucamine is also conveniently present in the solution. A small amount of an oxidant such as hydrogen peroxide may also be present to prevent the L-cystine from degrading to its thiol cysteine prior to administration.

To avoid any risk of nausea, L-cystine should be administered to a total daily dose of approximately 1.0 g or less. Higher doses produce a progressively greater risk of nausea in the patient, so that a preliminary test of the patient's tolerance may be advisable at such higher doses.

In the case of an oxidising agent and organic disulfide agent together, the agents may be administered simultaneously or sequentially. It may, for example, be found convenient to administer the oxidising agent first, to immobilise reducing enzymes of the patient's body, which could otherwise degrade the disulfide, then to administer the disulfide and then to follow this by administration of the microwave radiation immediately when the agents have reached the cancer site. This sequential administration may also be found desirable when the agents could otherwise mutually pre-react away from the cancer site or when the second agent has a rather short half-life in the blood-stream.

An oxidising agent such as cumene hydroperoxide or t-butyl hydroperoxide may conveniently be used in an aqueous solution at a concentration of up to approximately 1.5 g/l (e.g. from about 0.25 to 1 g/l, suitably about 0.4 g/l), enabling a daily injection volume between about 30 and 150 ml (e.g. about 60 to 120 ml), delivering a daily dose of up to approximately 50 mg.

At least some of the compositions described above are themselves novel and constitute further aspects of the invention.

In a further aspect, therefore, the invention provides a novel parenterally administrable pharmaceutical composition for use in a method of therapy according to the invention, comprising an aqueous solution of a non-toxic organic disulfide of general formula III, e.g. cystine or penicillamine disulfide (or a non-toxic salt thereof) at a concentration of up to approximately 20 g/l (e.g. approximately 10-20 g/l), together with an oxidising agent of general formula VI or VII, e.g. cumene hydroperoxide or t-butyl hydroperoxide at a concentration of up to approximately 1.5 g/l (e.g. approximately 1 g/l), optionally with a sulfoximine of general formula VIII, e.g. methionine sulfoximine.

In a still further aspect, the invention provides a novel kit of two or more parenterally administrable pharmaceutical compositions containing active agents, selected from compounds of general formulae III, VI, VII and VIII, for rapidly sequential administration in a method of therapy according to the invention, a first composition comprising an aqueous solution containing a first active agent selected from the agents as defined above, and a second composition comprising an aqueous solution containing a second active agent selected from the agents as defined above, with the proviso that at least one of said first and second active agents is a compound of general formula III ; the said first and second compositions being optionally together with further composition(s) and instructional literature for the therapy.

The compositions are prepared by standard mixing techniques that will be readily apparent to those skilled in this art. In the case of sparingly soluble active agents a suitable solubilising agent should first be dissolved in the aqueous medium.

After administration to the patient of the active agent(s) according to the invention, a period of time should pass to allow the cancer site(s) to become surrounded by the active agent(s), whereupon the microwave radiation should be administered without undue delay. The radiation should normally be begun to be administered between 1 and 15 minutes after intravenous administration of the active agent(s) to the patient.

To administer the microwave radiation, which covers at least the cancer site(s) or optionally the entire body of the patient if that is most convenient, the patient is brought close to a suitable number of microwave antennae (e.g. 3 or 4 antennae arranged to encircle the patient's body) and the antennae energised to transmit UHF microwave radiation. Normally an area of at least about 30 cm radius around each cancer site should be irradiated, and for this purpose the patient may conveniently be moved under the antennae.

The total antennae power should be less than about 8 kW (e.g. adjustable between about 3 and about 8 kW) in order to remain tolerable to the average patient. Patients may preferably be treated in two or more sessions, preferably at least 10 sessions, at daily intervals or every other or every third day for a treatment period of up to about four weeks. Preferably, each session day involves administration of the active agent followed quickly by from 1 to about 5 exposures (e.g. three exposures) to the microwave radiation, each exposure to the radiation lasting for about 5 to 30 minutes, with a 5 to 40 minute rest period between exposures. The total length of treatment on each session day will be dependent on the length of time over which the agent(s) remain at the cancer site or in the bloodstream without substantial loss, and the length of rests between exposures which the patient requires. Small bodies (e.g. children) can normally tolerate a radiation power of about 3 to 4 kW per session, with larger bodies around 6 to 8 kW. If too high a power is used the patient's body temperature will rise, with possibly dangerous consequences.

As mentioned above, the microwave radiation should be in the frequency range of about 400-450 MHz. More preferably, the frequency should be in the range of about 425-450 MHz, most preferably 432-436 MHz (e.g. about 434 MHz).

The antennae are suitably standard commercially available microwave antennae of a convenient size and shape to accommodate the shape of the patient's body. Folded dipole antennae are suitable, and these may preferably be provided with adjustable inductor coils in known manner to emit predominantly H-wave microwave radiation of wavelength between about 65 and 75 cm (most preferably about 69 cm). Alternatively, each antenna may consist of a single circular turn approximately 19 cm in diameter (the diameter being chosen to resonate at the frequency of the transmitter), the plane of the circular antenna being held substantially tangential to the curvature of the patient's body. Such a circular antenna will also emit substantially H-wave radiation towards the patient's body. While the presence of E-wave radiation will not necessarily hinder the efficacy of the radiation, it can lead to greater heating of the patient's skin, which is undesirable.

Each antenna is suitably connected to a UHF generator by a coaxial cable via circulators to prevent adverse interaction between antennae. Each generator may if desired be set at a slightly different frequency at or around a central frequency in the range already mentioned (preferably about 434 MHz).

All equipment within about 2 metres of the antennae should be of non-metallic materials such as wood or plastic, including for example the patient support table.

Tests have yielded the following data, which are included purely for ease of understanding of the present invention, and not for limitation of the scope of the invention:

### EXAMPLE

### Compositions and administration volumes

A first aqueous solution of cumene hydroperoxide is prepared by diluting 0.4 ml (0.4 g) of a commercially available cumene solution of cumene hydroperoxide (80% cumene hydroperoxide in cumene) into 1 litre of normal (0.9%) saline. A second aqueous solution of L-cystine is prepared by first dissolving 58 g of N-methyl-D-glucamine in 1 litre of normal (0.9%) saline and then dissolving in 16 g of L-cystine. 120 ml of the first solution is administered via intravenous drip (delivering an approximately 50 mg dose of cumene hydroperoxide), and as soon as that administration is complete it is followed by 60 ml of the second solution (delivering an approximately 1 g dose of cystine).

### Toxicity

No nausea after the i.v. injection. No immediate or late changes in blood pressure, renal or liver function, electrolytes or haematology have been seen.

### Contra-indications

Most previous cytotoxic chemotherapy, however long ago, which will have destroyed the insulating properties of normal tissue so that cancer and normal tissues will have become electrically indistinguishable.

### Case History

A case history will now be described, purely by way of illustration and example and for ease of understanding the present invention, but without limitation.

PH, an Asian male then aged 42, was examined by an Ear, Nose & Throat surgeon after complaining of a blockage in the right nostril, blood-stained nasal discharge and a foul taste and smell in the mouth of approximately three months duration. Inspection revealed a tumour, from which a biopsy was taken and a partial resection performed to clear a temporary airway. The pathology report showed the tumour to be composed of a poorly differentiated squamous carcinoma typical of primary nasopharyngeal cancer, which is common in Asia but has a poor long-term response to conventional therapies.

**First Treatment** soon after diagnosis was by full course of combined microwave and X-ray therapy, to a total dose of 6,000 rads, which produced a temporary response, but 4½ years later there was a recurrence (proved by biopsy).

**Second Treatment** then was by cytotoxic chemotherapy over three courses, which failed.

**Third Treatment** followed at a diet and meditation unit in China, including two further courses of cytotoxic chemotherapy, which still produced no response, and by this time (1 year after the recurrence) biopsies confirmed active cancer at several sites, including a 4 cm primary recurrence, huge bilateral neck metastatic cancers in many cervical lymph nodes and X-ray-visualised nodal deposits in the superior mediastinum.

**Fourth Treatment**, according to the present invention, was then undertaken, by intravenous injection of cumene hydroperoxide solution followed by L-cystine solution, and then followed immediately by three applications of 434 MHz UHF microwave electromagnetic radiation from the vertex of the skull to the xiphisternal junction. Each microwave treatment was from four antennae each energised about 0.8 kW (total power 3.2 kW), and each application of microwave radiation was for five minutes, separated by rest periods of 20 minutes. The treatment was repeated to a total of 15 times in a three-week period and the said three-week course was itself performed a further two times, the first two months later and the second a further four months later.

Tests one month after the end of the final course of treatment showed that all the cancer had disappeared and subsequent monitoring for a period of six years has shown no evidence of cancer at the primary site and no clinical or radiological evidence of metastases. Patient remains in normal health.

## Claims (Claims for the following Contracting State(s): AT, BE, CH, DE, DK, FR, GB, IE, IT, LI, LU, MC, NL, PT, SE)

1. Use of a non-toxic organic disulfide having the general formula:
R-S-S-R¹ (III)
in which R and R¹, which may be the same or different, are selected from alkyl groups which may optionally be mono- or poly-substituted, in the preparation of a composition or compositions for use in a method of cancer therapy in which there is administered to a patient an effective amount of said non-toxic disulfide, and while said disulfide is present at a cancer site of the patient there is administered to the cancer site an effective dose of microwave electromagnetic radiation of frequency in the range of about 400-450MHz.

2. A use according to claim 1, wherein there is also used an oxidising agent selected from organic peroxides and hydroperoxides of the general formulae:
R³-O-O-R⁴ (VI)
and
R⁵-O-OH (VII)
respectively, in which R³, R⁴ and R⁵, which may be the same or different, are alkyl groups which may optionally be mono- or poly-substituted.

3. A use according to claim 1, wherein there is also used a non-toxic organic sulfoximine having the general formula: 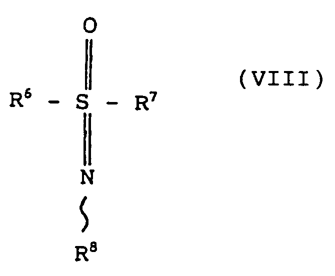 in which R⁶ and R⁷, which may be the same or different, are selected from alkyl groups which may optionally be mono- or poly-substituted and R⁸ is hydrogen or an alkyl group which may optionally be mono- or poly-substituted.

4. A use according to claim 1 wherein substituents of R and/or R¹ in general formula III are selected from halo, nitro, amino, C₁₋₄alkylamino, di-C₁₋₄ alkylamino, hydroxy, C₁₋₄alkoxy, carboxy, (C₁₋₄alkyl)-carbonyl, (C₁₋₄ alkoxy)-carbonyl, (C₁₋₄alkyl)-carbamoyl, carboxy-(C₁₋₄ alkyl)-carbamoyl, C₂₋₅alkanamido, salts and esters thereof, and derivatives thereof in which an alkyl portion of any substituent of R and/or R¹ is mono- or poly-substituted by NH₂ and/or CO₂H and/or salt derivatives thereof.

5. A use according to claim 2 wherein substituents of R³, R⁴ and/or R⁵ in general formulae VI and VII are selected from halo, nitro, aryl and substituted aryl.

6. A use according to claim 3 wherein substituents of R⁶, R⁷ and/or R⁸ in general formula VIII are selected from halo, nitro, amino, di-C₁₋₄alkylamino, hydroxy, c₁₋₄ alkoxy, carboxy, (C₁₋₄alkyl)-carbonyl, (C₁₋₄ alkoxy)-carbonyl, and salts and esters thereof.

7. A use according to any one of the preceding claims, wherein the non-toxic organic disulfide is in an aqueous solution at a concentration of up to approximately 20 g/l (e.g. approximately 10-20 g/l); and the oxidising agent, when present in the composition(s), is in an aqueous solution at a concentration of up to approximately 1.5 g/l (e.g. approximately 0.25 to 1 g/l).

8. A use according to any one of the preceding claims, wherein the organic disulfide is cystine, penicillamine disulfide or a non-toxic salt thereof, the oxidising agent, if present, is cumene hydroperoxide or t-butyl hydroperoxide, and the organic sulfoximine, if present, is methionine sulfoximine.

9. A use according to claim 8, wherein two sequentially administrable compositions are prepared, a first containing the agent to be administered first which is cumene hydroperoxide or t-butyl hydroperoxide and a second containing the agent to be administered subsequently which is L-cystine or penicillamine disulfide.

10. A use according to claim 2 or 9, wherein the composition(s) is/are in the form of dosage units for administration of cumene hydroperoxide or t-butyl hydroperoxide in a daily dose of up to about 50 mg.

11. A use according to claim 1 or 9, wherein the composition(s) is/are in the form of dosage units for administration of cystine or penicillamine disulfide in a daily dose of up to about 3 g.

12. A use according to any one of the preceding claims, wherein an organic alcohol (e.g. ethanol) is also used in the preparation of one or more of the compositions and/or an additional composition.

13. A parenterally administrable pharmaceutical composition for use in a method of cancer therapy, the composition comprising an aqueous solution of a non-toxic organic disulfide of general formula
R-S-S-R¹ (III)
together with an oxidising agent of general formula
R³-O-O-R⁴ (VI)
or
R⁵-O-OH (VII),
optionally with a non-toxic sulfoximine of general formula 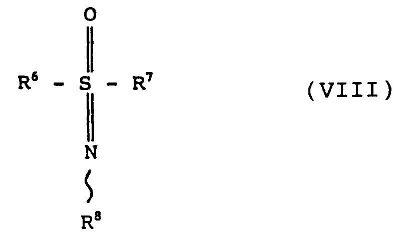 in which R to R⁷, which may be the same or different, are selected from alkyl groups which may optionally be mono-or poly-substituted and R⁸ is hydrogen or an alkyl group which may optionally be mono- or poly-substituted.

14. A kit of two or more parenterally administrable pharmaceutical compositions containing active agents selected from compounds of general formulae
R-S-S-R¹ (III)
R³-O-O-R⁴ (VI)
R⁵-O-OH (VII)
and 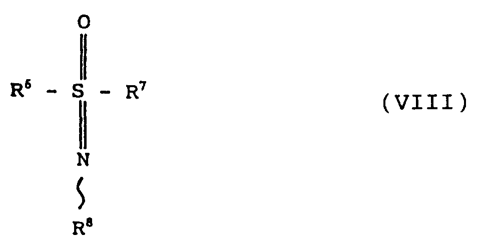 in which R to R⁷, which may be the same or different, are selected from alkyl groups which may optionally be mono-or poly-substituted and R⁸ is hydrogen or an alkyl group which may optionally be mono- or poly-substituted, for rapidly sequential administration in a method of cancer therapy, a first composition comprising an aqueous solution containing a first active agent selected from the agents as defined above, and a second composition comprising an aqueous solution containing a second active agent selected from the agents as defined above, with the proviso that at least one of said first and second active agents is a compound of general formula III ; the said first and second compositions being optionally together with further composition(s) and instructional literature for the therapy.

## Claims (Claims for the following Contracting State(s): ES, GR)

1. Use of a non-toxic organic disulfide having the general formula:
R-S-S-R¹ (III)
in which R and R¹, which may be the same or different, are selected from alkyl groups which may optionally be mono- or poly-substituted, in the preparation of a composition or compositions for use in a method of cancer therapy in which there is administered to a patient an effective amount of said non-toxic disulfide, and while said disulfide is present at a cancer site of the patient there is administered to the cancer site an effective dose of microwave electromagnetic radiation of frequency in the range of about 400-450MHz.

2. A use according to claim 1, wherein there is also used an oxidising agent selected from organic peroxides and hydroperoxides of the general formulae:
R³-O-O-R⁴ (VI)
and
R⁵-O-OH (VII)
respectively, in which R³, R⁴ and R⁵, which may be the same or different, are alkyl groups which may optionally be mono- or poly-substituted.

3. A use according to claim 1, wherein there is also used a non-toxic organic sulfoximine having the general formula: 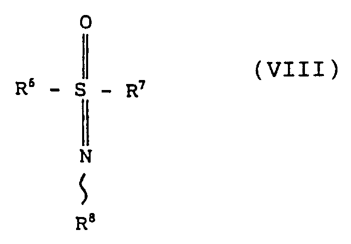 in which R⁶ and R⁷, which may be the same or different, are selected from alkyl groups which may optionally be mono- or poly-substituted and R⁸ is hydrogen or an alkyl group which may optionally be mono- or poly-substituted.

4. A use according to claim 1 wherein substituents of R and/or R¹ in general formula III are selected from halo, nitro, amino, C₁₋₄alkylamino, di-C₁₋₄ alkylamino, hydroxy, C₁₋₄alkoxy, carboxy, (C₁₋₄alkyl)-carbonyl, (C₁₋₄ alkoxy)-carbonyl, (C₁₋₄alkyl)-carbamoyl, carboxy-(C₁₋₄ alkyl)-carbamoyl, C₂₋₅alkanamido, salts and esters thereof, and derivatives thereof in which an alkyl portion of any substituent of R and/or R¹ is mono- or poly-substituted by NH₂ and/or CO₂H and/or salt derivatives thereof.

5. A use according to claim 2 wherein substituents of R³, R⁴ and/or R⁵ in general formulae VI and VII are selected from halo, nitro, aryl and substituted aryl.

6. A use according to claim 3 wherein substituents of R⁶, R⁷ and/or R⁸ in general formula VIII are selected from halo, nitro, amino, di-C₁₋₄alkylamino, hydroxy, C₁₋₄ alkoxy, carboxy, (C₁₋₄alkyl)-carbonyl, (C₁₋₄ alkoxy)-carbonyl, and salts and esters thereof.

7. A use according to any one of the preceding claims, wherein the non-toxic organic disulfide is in an aqueous solution at a concentration of up to approximately 20 g/l (e.g. approximately 10-20 g/l); and the oxidising agent, when present in the composition(s), is in an aqueous solution at a concentration of up to approximately 1.5 g/l (e.g. approximately 0.25 to 1 g/l).

8. A use according to any one of the preceding claims, wherein the organic disulfide is cystine, penicillamine disulfide or a non-toxic salt thereof, the oxidising agent, if present, is cumene hydroperoxide or t-butyl hydroperoxide, and the organic sulfoximine, if present, is methionine sulfoximine.

9. A use according to claim 8, wherein two sequentially administrable compositions are prepared, a first containing the agent to be administered first which is cumene hydroperoxide or t-butyl hydroperoxide and a second containing the agent to be administered subsequently which is L-cystine or penicillamine disulfide.

10. A use according to claim 2 or 9, wherein the composition(s) is/are in the form of dosage units for administration of cumene hydroperoxide or t-butyl hydroperoxide in a daily dose of up to about 50 mg.

11. A use according to claim 1 or 9, wherein the composition(s) is/are in the form of dosage units for administration of cystine or penicillamine disulfide in a daily dose of up to about 3 g.

12. A use according to any one of the preceding claims, wherein an organic alcohol (e.g. ethanol) is also used in the preparation of one or more of the compositions and/or an additional composition.

13. A method for preparing a parenterally administrable pharmaceutical composition for use in a method of cancer therapy, the composition comprising an aqueous solution of a non-toxic organic disulfide of general formula
R-S-S-R¹ (III)
together with an oxidising agent of general formula
R³-O-O-R⁴ (VI)
or
R⁵-O-OH (VII),
optionally with a sulfoximine of general formula 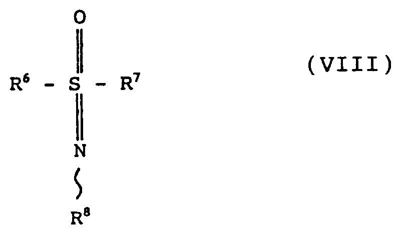 in which R to R⁷, which may be the same or different, are selected from alkyl groups which may optionally be monoor poly-substituted and R⁸ is hydrogen or an alkyl group which may optionally be mono- or poly-substituted, and the preparative method comprising dissolving the said non-toxic organic disulfide and the said oxidising agent, optionally with the said sulfoximine, and any desired additional components, in a suitable pharmacologically acceptable aqueous medium.

14. A method for preparing a kit of two or more parenterally administrable pharmaceutical compositions containing active agents selected from compounds of general formulae
R-S-S-R¹ (III)
R³-O-O-R⁴ (VI)
R⁵-O-OH (VII)
and 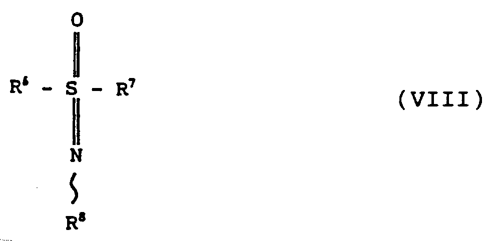 in which R to R⁷, which may be the same or different, are selected from alkyl groups which may optionally be mono-or poly-substituted and R⁸ is hydrogen or an alkyl group which may optionally be mono- or poly-substituted, for rapidly sequential administration in a method of cancer therapy, a first composition comprising an aqueous solution containing a first active agent selected from the agents as defined above, and a second composition comprising an aqueous solution containing a second active agent selected from the agents as defined above, with the proviso that at least one of said first and second active agents is a compound of general formula III ; the said first and second compositions being optionally together with further composition(s) and instructional literature for the therapy, and the preparative method comprising dissolving the said first active agent and any desired additional components in a first suitable pharmacologically acceptable aqueous medium to prepare the said first composition, and dissolving the said second active agent and any desired additional components in a second suitable pharmacologically acceptable aqueous medium to prepare the said second composition.

## Patentansprüche (Patentansprüche für folgende(n) Vertragsstaat(en): AT, BE, CH, DE, DK, FR, GB, IE, IT, LI, LU, MC, NL, PT, SE)

1. Verwendung eines nicht-toxischen organischen Disulfids der folgenden allgemeinen Formel:
R-S-S-R¹ (III)
worin R und R¹, die gleich oder verschieden sein können, ausgewählt sind unter Alkylgruppen, die gewünschtenfalls mono- oder polysubstituiert sein können, bei der Herstellung einer Zusammensetzung oder von Zusammensetzungen zur Anwendung in einem Verfahren der Krebstherapie, bei dem einem Patienten eine wirksame Menge dieses nicht-toxischen Disulfids verabreicht wird und bei dem, während dieses Disulfid an dem Karzinomort des Patienten vorhanden ist, an den Karzinomort eine wirksame Dosis einer elektromagnetischen Mikrowellenstrahlung mit einer Frequenz im Bereich von etwa 400 bis 450 MHz verabreicht wird.

2. Verwendung nach Anspruch 1, bei der außerdem ein oxidierendes Agens verwendet wird, das ausgewählt ist unter organischen Peroxiden und Hydroperoxiden der allgemeinen Formeln:
R³-O-O-R⁴ (VI)
und
R⁵-O-OH (VII)
worin R³, R⁴ und R⁵, die gleich oder verschieden sein können, Alkylgruppen darstellen, die gewünschtenfalls mono- oder polysubstituiert sein können.

3. Verwendung nach Anspruch 1, bei der auch ein nicht-toxisches organisches Sulfoximin der folgenden allgemeinen Formel: 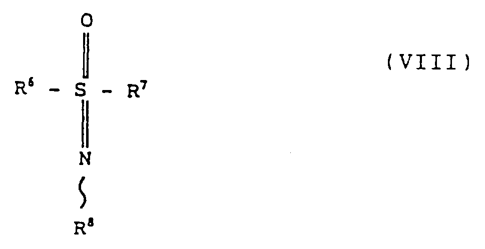 worin R⁶ und R⁷, die gleich oder verschieden sein können, ausgewählt sind unter Alkylgruppen, die gewünschtenfalls mono- oder polysubstituiert sein können, und R⁸ für Wasserstoff oder eine Alkylgruppe steht, die gewünschtenfalls mono- oder polysubstituiert sein kann, eingesetzt wird.

4. Verwendung nach Anspruch 1, bei der die Substituenten R und/oder R¹ in der allgemeinen Formel III ausgewählt sind unter Halogen, Nitro, Amino, C₁₋₄Alkylamino, di-C₁₋₄Alkylamino, Hydroxy, C₁₋₄Alkoxy, Carboxy, (C₁₋₄Alkyl)-carbonyl, (C₁₋₄Alkoxy)-carbonyl, (C₁₋₄Alkyl)-carbamoyl, Carboxy-(C₁₋₄alkyl)-carbamoyl, C₂₋₅Alkanamido, Salzen und Estern davon sowie den Derivaten davon, bei denen eine Alkyleinheit eines beliebigen Substituenten von R und/oder R¹ durch NH₂ oder CO₂H und/oder den Salzderivaten davon.

5. Verwendung nach Anspruch 2, bei der die Substituenten von R³, R⁴ und/oder R⁵ in den allgemeinen Formel VI und VII ausgewählt sind unter Halogen, Nitro, Aryl und substituiertem Aryl.

6. Verwendung nach Anspruch 3, bei der die Substituenten von R⁶, R⁷ und/oder R⁸ in der allgemeinen Formel VIII ausgewählt sind unter Halogen, Nitro, Amino, di-C₁₋₄Alkylamino, Hydroxy, C₁₋₄Alkoxy, Carboxy, (C₁₋₄Alkyl)-carbonyl, (C₁₋₄Alkoxy)-carbonyl und den Salzen und Estern davon.

7. Verwendung nach einem der vorhergehenden Ansprüche, bei der das nicht-toxische organische Disulfid in einer wässrigen Lösung in einer Konzentration von bis zu etwa 20 g/l (z.B. in etwa 10 bis 20 g/l) vorhanden ist und das oxidierende Agens, wenn es in der Zusammensetzung oder in den Zusammensetzungen vorhanden ist, in einer Konzentration von bis zu etwa 1,5 g/l (z.B. in etwa 0,25 bis 1 g/l) in einer wässrigen Lösung vorhanden ist.

8. Verwendung nach einem der vorhergehenden Ansprüche, bei der das organische Disulfid Cystin, Penicillamindisulfid oder ein nicht-toxisches Salz davon, das oxidierende Agens, sofern es vorhanden ist, Cumenhydroperoxid oder t-Butylhydroperoxid und das organische Sulfoximin, sofern es vorhanden ist, Methioninsulfoximin sind.

9. Verwendung nach Anspruch 8, bei der zwei nacheinander verabreichbare Zusammensetzungen hergestellt werden, wobei eine erste das zuerst zu verabreichende Agens enthält, bei dem es sich um Cumenhydroperoxid oder t-Butylhydroperoxid handelt, und eine zweite das im Anschluß daran zu verabreichende Agens enthält, bei dem es sich um L-Cystin oder Penicillamindisulfid handelt.

10. Verwendung nach Anspruch 2 oder 9, bei der die Zusammensetzung(en) in Form von Dosierungseinheiten zur Verabreichung von Cumenhydroperoxid oder t-Butylhydroperoxid in einer Tagesdosis von bis zu etwa 50 mg vorliegt/ vorliegen.

11. Verwendung nach Anspruch 1 oder 9, bei der die Zusammensetzung(en) in Form von Dosierungseinheiten zur Verabreichung von Cystin oder Penicillamindisulfid in einer Tagesdosis von bis zu etwa 3 g vorliegt/vorliegen.

12. Verwendung nach einem der vorhergehenden Ansprüche, bei der ein organischer Alkohol (z.B. Ethanol) ebenfalls bei der Herstellung von einer oder mehreren der Zusammensetzungen und/oder einer weiteren Zusammensetzung verwendet wird.

13. Parenteral verabreichbare pharmazeutische Zusammensetzung zur Anwendung in einem Verfahren der Krebstherapie, wobei die Zusammensetzung eine wässrige Lösung eines nicht-toxischen organischen Disulfids der allgemeinen Formel
R-S-S-R¹ (III)
zusammen mit einem oxidierenden Agens der allgemeinen Formel
R³-O-O-R⁴ (VI)
oder
R⁵-O-OH (VII)
gewünschtenfalls zusammen mit einem nicht-toxischen Sulfoximin der allgemeinen Formel 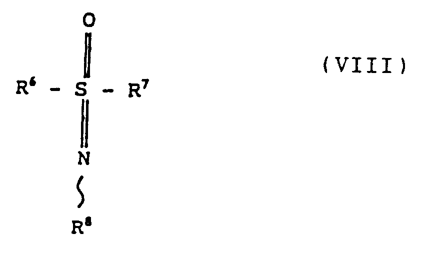 worin R bis R⁷, die gleich oder verschieden sein können, ausgewählt sind unter Alkylgruppen, die gewünschtenfalls mono- oder polysubstituiert sein können, und R⁸ für Wasserstoff oder eine Alkylgruppe steht, die gewünschtenfalls mono- oder polysubstituiert sein kann, aufweist.

14. Kit aus zwei oder mehr parenteral verabreichbaren pharmazeutischen Zusammensetzungen, die Wirkstoffe enthalten, die ausgewählt sind unter den Verbindungen der allgemeinen Formeln
R-S-S-R¹ (III)
R³-O-O-R⁴ (VI)
R⁵-O-OH (VII)
und 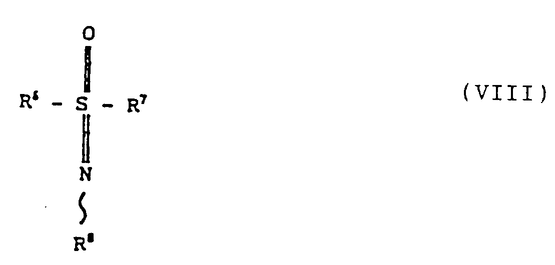 worin R bis R⁷, die gleich oder verschieden sein können, ausgewählt sind unter Alkylgruppen, die gewünschtenfalls mono- oder polysubstituiert sein können, und R⁸ für Wasserstoff oder eine Alkylgruppe steht, die gewünschtenfalls mono- oder polysubstituiert sein kann, zur schnell aufeinanderfolgenden Verabreichung in einem Verfahren zur Krebstherapie, wobei eine erste Zusammensetzung eine wässrige Lösung aufweist, welche einen ersten Wirkstoff enthält, der ausgewählt ist unter den oben definierten Wirkstoffen, und wobei eine zweite Zusammensetzung eine wässrige Lösung aufweist, die einen zweiten Wirkstoff enthält, der aus den oben definierten Agenzien ausgewählt ist, mit der Maßgabe, daß mindestens einer der ersten und zweiten Wirkstoffe eine Verbindung der allgemeinen Formel III ist, wobei die erste und zweite Zusammensetzung gewünschtenfalls zusammen mit einer weiteren oder weiteren Zusammensetzung(en) vorliegen, und Anweisungsliteratur für die Therapie.

## Patentansprüche (Patentansprüche für folgende(n) Vertragsstaat(en): ES, GR)

1. Verwendung eines nicht-toxischen organischen Disulfids der folgenden allgemeinen Formel:
R-S-S-R¹ (III)
worin R und R¹, die gleich oder verschieden sein können, ausgewählt sind unter Alkylgruppen, die gewünschtenfalls mono- oder polysubstituiert sein können, bei der Herstellung einer Zusammensetzung oder von Zusammensetzungen zur Anwendung in einem Verfahren der Krebstherapie, bei dem einem Patienten eine wirksame Menge dieses nicht-toxischen Disulfids verabreicht wird und bei dem, während dieses Disulfid an dem Karzinomort des Patienten vorhanden ist, an den Karzinomort eine wirksame Dosis einer elektromagnetischen Mikrowellenstrahlung mit einer Frequenz im Bereich von etwa 400 bis 450 MHz verabreicht wird.

2. Verwendung nach Anspruch 1, bei der außerdem ein oxidierendes Agens verwendet wird, das ausgewählt ist unter organischen Peroxiden und Hydroperoxiden der allgemeinen Formeln:
R³-O-O-R⁴ (VI)
und
R⁵-O-OH (VII)
worin R³, R⁴ und R⁵, die gleich oder verschieden sein können, Alkylgruppen darstellen, die gewünschtenfalls mono- oder polysubstituiert sein können.

3. Verwendung nach Anspruch 1, bei der auch ein nicht-toxisches organisches Sulfoximin der folgenden allgemeinen Formel: 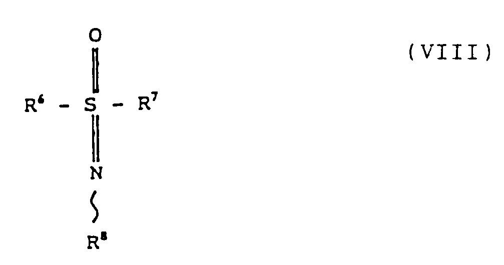 worin R⁶ und R⁷, die gleich oder verschieden sein können, ausgewählt sind unter Alkylgruppen, die gewünschtenfalls mono- oder polysubstituiert sein können, und R⁸ für Wasserstoff oder eine Alkylgruppe steht, die gewünschtenfalls mono- oder polysubstituiert sein kann, eingesetzt wird.

4. Verwendung nach Anspruch 1, bei der die Substituenten R und/oder R¹ in der allgemeinen Formel III ausgewählt sind unter Halogen, Nitro, Amino, C₁₋₄Alkylamino, di-C₁₋₄Alkylamino, Hydroxy, C₁₋₄Alkoxy, Carboxy, (C₁₋₄Alkyl)-carbonyl, (C₁₋₄Alkoxy)-carbonyl, (C₁₋₄Alkyl)-carbamoyl, Carboxy-(C₁₋₄alkyl)-carbamoyl, C₂₋₅Alkanamido, Salzen und Estern davon sowie den Derivaten davon, bei denen eine Alkyleinheit eines beliebigen Substituenten von R und/oder R¹ durch NH₂ oder CO₂H und/oder den Salzderivaten davon.

5. Verwendung nach Anspruch 2, bei der die Substituenten von R³, R⁴ und/oder R⁵ in den allgemeinen Formel VI und VII ausgewählt sind unter Halogen, Nitro, Aryl und substituiertem Aryl.

6. Verwendung nach Anspruch 3, bei der die Substituenten von R⁶, R⁷ und/oder R⁸ in der allgemeinen Formel VIII ausgewählt sind unter Halogen, Nitro, Amino, di-C₁₋₄Alkylamino, Hydroxy, C₁₋₄Alkoxy, Carboxy, (C₁₋₄Alkyl)-carbonyl, (C₁₋₄Alkoxy)-carbonyl und den Salzen und Estern davon.

7. Verwendung nach einem der vorhergehenden Ansprüche, bei der das nicht-toxische organische Disulfid in einer wässrigen Lösung in einer Konzentration von bis zu etwa 20 g/l (z.B. in etwa 10 bis 20 g/l) vorhanden ist und das oxidierende Agens, wenn es in der Zusammensetzung oder in den Zusammensetzungen vorhanden ist, in einer Konzentration von bis zu etwa 1,5 g/l (z.B. in etwa 0,25 bis 1 g/l) in einer wässrigen Lösung vorhanden ist.

8. Verwendung nach einem der vorhergehenden Ansprüche, bei der das organische Disulfid Cystin, Penicillamindisulfid oder ein nicht-toxisches Salz davon, das oxidierende Agens, sofern es vorhanden ist, Cumenhydroperoxid oder t-Butylhydroperoxid und das organische Sulfoximin, sofern es vorhanden ist, Methioninsulfoximin sind.

9. Verwendung nach Anspruch 8, bei der zwei nacheinander verabreichbare Zusammensetzungen hergestellt werden, wobei eine erste das zuerst zu verabreichende Agens enthält, bei dem es sich um Cumenhydroperoxid oder t-Butylhydroperoxid handelt, und eine zweite das im Anschluß daran zu verabreichende Agens enthält, bei dem es sich um L-Cystin oder Penicillamindisulfid handelt.

10. Verwendung nach Anspruch 2 oder 9, bei der die Zusammensetzung(en) in Form von Dosierungseinheiten zur Verabreichung von Cumenhydroperoxid oder t-Butylhydroperoxid in einer Tagesdosis von bis zu etwa 50 mg vorliegt/vorliegen.

11. Verwendung nach Anspruch 1 oder 9, bei der die Zusammensetzung(en) in Form von Dosierungseinheiten zur Verabreichung von Cystin oder Penicillamindisulfid in einer Tagesdosis von bis zu etwa 3 g vorliegt/vorliegen.

12. Verwendung nach einem der vorhergehenden Ansprüche, bei der ein organischer Alkohol (z.B. Ethanol) ebenfalls bei der Herstellung von einer oder mehreren der Zusammensetzungen und/oder einer weiteren Zusammensetzung verwendet wird.

13. Parenteral verabreichbare pharmazeutische Zusammensetzung zur Anwendung in einem Verfahren der Krebstherapie, wobei die Zusammensetzung eine wässrige Lösung eines nicht-toxischen organischen Disulfids der allgemeinen Formel
R-S-S-R¹ (III)
zusammen mit einem oxidierenden Agens der allgemeinen Formel
R³-O-O-R⁴ (VI)
oder
R⁵-O-OH (VII)
gewünschtenfalls zusammen mit einem nicht-toxischen Sulfoximin der allgemeinen Formel 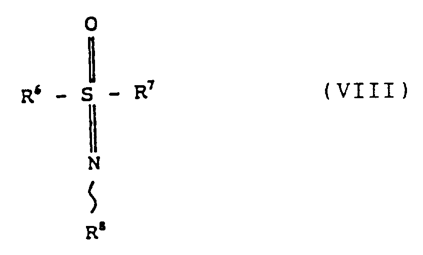 worin R bis R⁷, die gleich oder verschieden sein können, ausgewählt sind unter Alkylgruppen, die gewünschtenfalls mono- oder polysubstituiert sein können, und R⁸ für Wasserstoff oder eine Alkylgruppe steht, die gewünschtenfalls mono- oder polysubstituiert ist, und wobei das Herstellungsverfahren die Auflösung dieses nicht-toxischen organischen Disulfids und des oxidierenden Agens gewünschtenfalls mit dem Sulfoximin, und jedwedem gewünschten weiteren Bestandteil in einem geeigneten pharmakologisch akzeptierbaren wässrigen Medium umfaßt.

14. Verfahren zur Herstellung eines Kits aus zwei oder mehr parenteral verabreichbaren pharmazeutischen Zusammensetzungen, die Wirkstoffe enthalten, die ausgewählt sind unter den Verbindungen der allgemeinen Formeln
R-S-S-R¹ (III)
R³-O-O-R⁴ (VI)
R⁵-O-OH (VII)
und 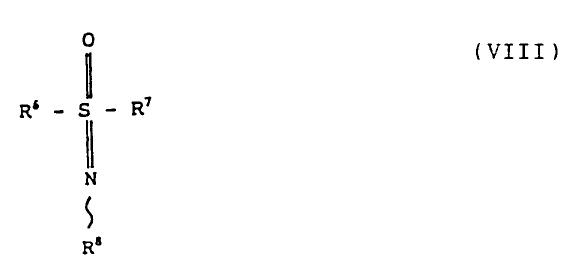 worin R bis R⁷, die gleich oder verschieden sein können, ausgewählt sind unter Alkylgruppen, die gewünschtenfalls mono- oder polysubstituiert sein können, und R⁸ für Wasserstoff oder eine Alkylgruppe steht, die gewünschtenfalls mono- oder polysubstituiert sein kann, zur schnell aufeinanderfolgenden Verabreichung in einem Verfahren zur Krebstherapie, wobei eine erste Zusammensetzung eine wässrige Lösung aufweist, welche einen ersten Wirkstoff enthält, der ausgewählt ist unter den oben definierten Wirkstoffen, und wobei eine zweite Zusammensetzung eine wässrige Lösung aufweist, die einen zweiten Wirkstoff enthält, der aus den oben definierten Agenzien ausgewählt ist, mit der Maßgabe, daß mindestens einer der ersten und zweiten Wirkstoffe eine Verbindung der allgemeinen Formel III ist, wobei die erste und zweite Zusammensetzung gewünschtenfalls zusammen mit einer weiteren oder weiteren Zusammensetzung(en) vorliegen und Anweisungsliteratur für die Therapie, und wobei das Herstellungsverfahren das Auflösen des ersten aktiven Agens und jedes gewünschten weiteren Bestandteils in einem ersten geeigneten, pharmakologisch akzeptierbaren wässrigen Medium zur Herstellung dieser ersten Zusammensetzung und die Auflösung des zweiten aktiven Agens und jedes gewünschten weiteren Bestandteils in einem zweiten geeigneten pharmakologisch akzeptierbaren wässrigen Medium zur Herstellung dieser zweiten Zusammensetzung umfaßt.

## Revendications (Revendications pour l'(les) Etat(s) contractant(s) suivant(s): AT, BE, CH, DE, DK, FR, GB, IE, IT, LI, LU, MC, NL, PT, SE)

1. Utilisation d'un disulfure organique non toxique de formule générale :
R - S - S - R¹ (III)
dans laquelle R et R¹, qui peuvent être identiques ou différents, représentent des groupes alkyles qui peuvent être optionnellement mono- ou poly-substitués,
dans la préparation d'une composition ou de compositions destinée(s) à être utilisée(s) dans une méthode de thérapie cancéreuse dans laquelle on administre à un patient une quantité effective dudit disulfure non toxique, et tandis que ledit disulfure est présent à un site cancéreux du patient on administre audit site cancéreux une dose effective d'une radiation électromagnétique à micro-ondes de fréquence située aux environs de 400-450 MHz.

2. Utilisation selon la revendication 1, dans laquelle on utilise également un agent oxydant choisi parmi les peroxydes et les hydroperoxydes organiques de formules générales respectives :
R³ - O - O - R⁴ (VI)
et
R⁵ - O - 0H (VII),
dans lesquelles R³, R⁴ et R⁵, qui peuvent être identiques ou différents, sont des groupes alkyles qui peuvent être optionnellement mono- ou poly-substitués.

3. Utilisation selon la revendication 1, dans laquelle on utilise également une sulfoximine organique non toxique de formule générale : 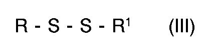 dans laquelle R⁶ et R⁷, qui peuvent être identiques ou différents, représentent des groupes alkyles qui peuvent être optionnellement mono- ou poly-substitués et R⁸ est un atome d'hydrogène ou un groupe alkyle qui peut être optionnellement mono- ou poly-substitué.

4. Utilisation selon la revendication 1 dans laquelle les substituants de R et/ou R¹ dans la formule générale III sont choisis parmi les substituants halo, nitro, amino, C₁₋ ₄alkylamino, di-C₁₋₄alkylamino, hydroxy, C₁₋₄alkoxy, carboxy, (C₁₋₄alkyl)-carbonyle, (C₁₋₄ alkoxy)-carbonyle, (C₁₋₄alkyl)-carbamoyle, carboxy-(C₁₋₄ alkyl)-carbamoyle, C₂₋₅alkanamido, leurs sels et esters, ainsi que leurs dérivés dans lesquels une partie alkyle d'un substituant quelconque de R et/ou R¹ est mono- ou poly-substituée par NH₂ et/ou CO₂H et/ou des sels en dérivant.

5. Utilisation selon la revendication 2 dans laquelle les substituants de R³, R⁴ et/ou R⁵ dans les formules générales VI et VII sont choisis parmi les substituants halo, nitro, aryle et aryle substitué.

6. Utilisation selon la revendication 3 dans laquelle les substituants de R⁶, R⁷ et/ou R⁸ dans la formule générale VIII sont choisis parmi les substituants halo, nitro, amino, di-C₁₋₄alkylamino, hydroxy, C₁₋₄alkoxy, carboxy, (C₁₋₄alkyl)-carbonyle, (C₁₋₄alkoxy) -carbonyle, et leurs sels et esters.

7. Utilisation selon l'une quelconque des revendications précédentes, dans laquelle le disulfure organique non toxique est en solution aqueuse à une concentration allant jusqu'à environ 20g/l (par exemple environ 10 à 20 g/l), et l'agent oxydant, s'il est présent dans la composition, est en solution aqueuse à une concentration allant jusqu'à environ 1,5 g/l (par exemple environ 0,25 à 1 g/l).

8. Utilisation selon l'une quelconque des revendications précédentes, dans laquelle le disulfure organique est la cystine, le disulfure de pénicillamine ou un de leurs sels non toxiques ; l'agent oxydant, s'il est présent, est l'hydroperoxyde de cumène ou l'hydroperoxyde de t-butyle ; et la sulfoximine organique, si elle est présente, est la sulfoximine de méthionine.

9. Utilisation selon la revendication 8, dans laquelle deux compositions séquentiellement administrables sont préparées, une première contenant l'agent à administrer en premier qui est l'hydroperoxyde de cumène ou l'hydroperoxide de t-butyle, et une seconde contenant l'agent à administrer ultérieurement, qui est la L-cystine ou le disulfure de pénicillamine.

10. Utilisation selon la revendication 2 ou la revendication 9, dans laquelle la(les) composition(s) est/sont sous forme de doses unitaires pour l'administration d'hydroperoxyde de cumène ou d'hydroperoxyde de t-butyle à une dose journalière allant jusqu'à environ 50 mg.

11. Utilisation selon la revendication 1 ou la revendication 9, dans laquelle la (les) composition(s) est/sont sous forme de doses unitaires pour l'administration de cystine ou de disulfure de pénicillamine à une dose journalière allant jusqu'à environ 3g.

12. Utilisation selon l'une quelconque des revendications précédentes, dans laquelle un alcool organique (par exemple l'éthanol) est également utilisé dans la préparation d'au moins une des compositions et/ou d'une composition additionnelle.

13. Composition pharmaceutique administrable par voie parentérale, destinée à être utilisée dans une méthode de thérapie cancéreuse, ladite composition comprenant une solution aqueuse d'un disulfure organique de formule générale
R - S - S - R¹ (III)
et d'un agent oxydant de formule générale
R³ - O - O - R⁴ (VI)
ou
R⁵ - O - OH (VII),
optionnellement avec une sulfoximine non toxique de formule générale 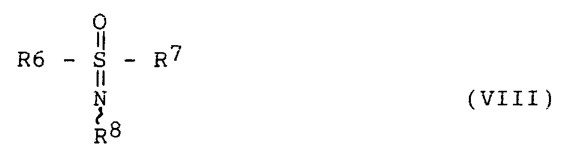 dans lesquelles R à R⁷, qui peuvent être identiques ou différents, représentent des groupes alkyles qui peuvent être optionnellement mono- ou poly-substitués et R⁸ représente un atome d'hydrogène ou un groupe alkyle qui peut être optionnellement mono- ou poly-substitué.

14. Kit de deux, ou plus, compositions pharmaceutiques administrables par voie parentérale, contenant des agents actifs choisis parmi les composés de formules générales
R - S - S - R¹ (III)
R3 - O - O - R⁴ (VI)
R⁵ - O - OH (VII)
et 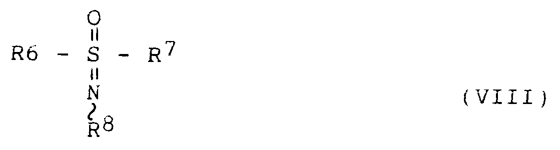 dans lesquelles R à R⁷, qui peuvent être identiques ou différents, représentent des groupes alkyles qui peuvent être optionnellement mono- ou poly-substitués et R⁸ représente un atome d'hydrogène ou un groupe alkyle qui peut être optionnellement mono- ou poly-substitué,
pour administration rapidement séquentielle dans une méthode de thérapie cancéreuse, une première composition comprenant une solution aqueuse contenant un premier agent actif choisi parmi les agents définis ci-dessus, et une deuxième composition comprenant une solution aqueuse contenant un deuxième agent actif choisi parmi les agents définis ci-dessus, avec la condition qu'au moins l'un desdits premier et deuxième agents actifs est un composé de formule générale (III) ; lesdites première et deuxième compositions étant optionnellement accompagnées d'un autre (d'autres) composition(s) et d'une notice d'instructions pour la thérapie.

## Revendications (Revendications pour l'(les) Etat(s) contractant(s) suivant(s): ES, GR)

1. Utilisation d'un disulfure organique non toxique de formule générale :
R - S - S - R¹ (III)
dans laquelle R et R¹, qui peuvent être identiques ou différents, représentent des groupes alkyles qui peuvent être optionnellement mono- ou poly-substitués,
dans la préparation d'une composition ou de compositions destinée(s) à être utilisée(s) dans une méthode de thérapie cancéreuse dans laquelle on administre à un patient une quantité effective dudit disulfure non toxique, et tandis que ledit disulfure est présent à un site cancéreux du patient on administre audit site cancéreux une dose effective d'une radiation électromagnétique à micro-ondes de fréquence située aux environs de 400-450 MHz.

2. Utilisation selon la revendication 1, dans laquelle on utilise également un agent oxydant choisi parmi les peroxydes et les hydroperoxydes organiques de formules générales respectives :
R³ - O - O - R⁴ (VI)
et
R⁵ - O - 0H (VII),
dans lesquelles R³, R⁴ et R⁵, qui peuvent être identiques ou différents, sont des groupes alkyles qui peuvent être optionnellement mono- ou poly-substitués.

3. Utilisation selon la revendication 1, dans laquelle on utilise également une sulfoximine organique non toxique de formule générale : 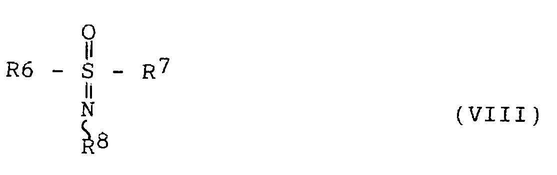 ans laquelle R⁶ et R⁷, qui peuvent être identiques ou différents, représentent des groupes alkyles qui peuvent être optionnellement mono- ou poly-substitués et R⁸ est un atome d'hydrogène ou un groupe alkyle qui peut être optionnellement mono- ou poly-substitué.

4. Utilisation selon la revendication 1 dans laquelle les substituants de R et/ou R¹ dans la formule générale III sont choisis parmi les substituants halo, nitro, amino, C₁₋ ₄alkylamino, di-C₁₋₄alkylamino, hydroxy, C₁₋₄alkoxy, carboxy, (C₁₋₄alkyl)-carbonyle, (C₁₋₄ alkoxy)-carbonyle, (C₁₋₄alkyl)-carbamoyle, carboxy-(C₁₋₄ alkyl)-carbamoyle, C₂₋₅alkanamido, leurs sels et esters, ainsi que leurs dérivés dans lesquels une partie alkyle d'un substituant quelconque de R et/ou R¹ est mono- ou poly-substituée par NH₂ et/ou CO₂H et/ou des sels en dérivant.

5. Utilisation selon la revendication 2 dans laquelle les substituants de R³, R⁴ et/ou R⁵ dans les formules générales VI et VII sont choisis parmi les substituants halo, nitro, aryle et aryle substitué.

6. Utilisation selon la revendication 3 dans laquelle les substituants de R⁶, R⁷ et/ou R⁸ dans la formule générale VIII sont choisis parmi les substituants halo, nitro, amino, di-C₁₋₄alkylamino, hydroxy, C₁₋₄alkoxy, carboxy, (C₁₋₄alkyl)-carbonyle, (C₁₋₄alkoxy) -carbonyle, et leurs sels et esters.

7. Utilisation selon l'une quelconque des revendications précédentes, dans laquelle le disulfure organique non toxique est en solution aqueuse à une concentration allant jusqu'à environ 20g/l (par exemple environ 10 à 20 g/l), et l'agent oxydant, s'il est présent dans la composition, est en solution aqueuse à une concentration allant jusqu'à environ 1,5 g/l (par exemple environ 0,25 à 1 g/l).

8. Utilisation selon l'une quelconque des revendications précédentes, dans laquelle le disulfure organique est la cystine, le disulfure de pénicillamine ou un de leurs sels non toxiques ; l'agent oxydant, s'il est présent, est l'hydroperoxyde de cumène ou l'hydroperoxyde de t-butyle ; et la sulfoximine organique, si elle est présente, est la sulfoximine de méthionine.

9. Utilisation selon la revendication 8, dans laquelle deux compositions séquentiellement administrables sont préparées, une première contenant l'agent à administrer en premier qui est l'hydroperoxyde de cumène ou l'hydroperoxide de t-butyle, et une seconde contenant l'agent à administrer ultérieurement, qui est la L-cystine ou le disulfure de pénicillamine.

10. Utilisation selon la revendication 2 ou la revendication 9, dans laquelle la(les) composition(s) est/sont sous forme de doses unitaires pour l'administration d'hydroperoxyde de cumène ou d'hydroperoxyde de t-butyle à une dose journalière allant jusqu'à environ 50 mg.

11. Utilisation selon la revendication 1 ou la revendication 9, dans laquelle la (les) composition(s) est/sont sous forme de doses unitaires pour l'administration de cystine ou de disulfure de pénicillamine à une dose journalière allant jusqu'à environ 3g.

12. Utilisation selon l'une quelconque des revendications précédentes, dans laquelle un alcool organique (par exemple l'éthanol) est également utilisé dans la préparation d'au moins une des compositions et/ou d'une composition additionnelle.

13. Méthode de préparation d'une composition pharmaceutique administrable par voie parentérale, destinée à être utilisée dans une méthode de thérapie cancéreuse, ladite composition comprenant une solution aqueuse d'un disulfure organique de formule générale
R - S - S - R¹ (III)
et d'un agent oxydant de formule générale
R³ - O - O - R⁴ (VI)
ou
R⁵ - O - OH (VII),
optionnellement avec une sulfoximine de formule générale 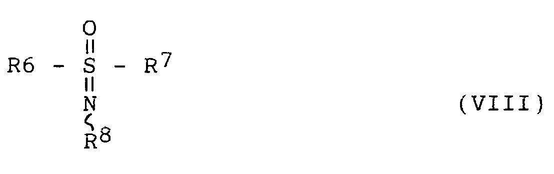 dans lesquelles R à R⁷, qui peuvent être identiques ou différents, représentent des groupes alkyles qui peuvent être optionnellement mono- ou poly-substitués et R⁸ représente un atome d'hydrogène ou un groupe alkyle qui peut être optionnellement mono- ou poly-substitué, et la méthode de préparation consistant à dissoudre ledit disulfure organique et ledit agent oxydant, optionnellement avec ladite sulfoximine et tout composé additionnel souhaitable, dans un milieu aqueux approprié pharmacologiquement acceptable.

14. Méthode de préparation d'un kit de deux, ou plus, compositions pharmaceutiques administrables par voie parentérale, contenant des agents actifs choisis parmi les composés de formules générales
R - S - S - R¹ (III)
R3 - O - O - R⁴ (VI)
R⁵ - O - OH (VII)
et 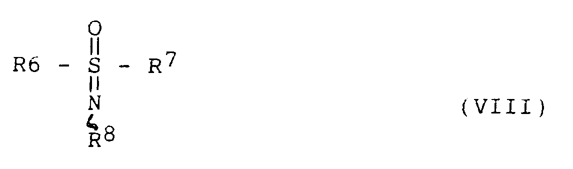 dans lesquelles R à R⁷, qui peuvent être identiques ou différents, représentent des groupes alkyles qui peuvent être optionnellement mono- ou poly-substitués et R⁸ représente un atome d'hydrogène ou un groupe alkyle qui peut être optionnellement mono- ou poly-substitué,
pour administration rapidement séquentielle dans une méthode de thérapie cancéreuse, une première composition comprenant une solution aqueuse contenant un premier agent actif choisi parmi les agents définis ci-dessus, et une deuxième composition comprenant une solution aqueuse contenant un deuxième agent actif choisi parmi les agents définis ci-dessus, avec la condition qu'au moins l'un desdits premier et deuxième agents actifs est un composé de formule générale (III) ; lesdites première et deuxième compositions étant optionnellement accompagnées d'un autre (d'autres) composition(s) et d'une notice d'instructions pour la thérapie ; et la méthode de préparation consistant à dissoudre ledit premier agent actif et tout composé additionnel souhaitable dans un premier milieu aqueux approprié pharmacologiquement acceptable afin de préparer ladite première composition, et à dissoudre ledit second agent actif et tout composé additionnel souhaitable dans un second milieu aqueux approprié pharmacologiquement acceptable afin de préparer ladite seconde composition.
